# EUROPÄISCHE PATENTSCHRIFT

(11) **EP 2 794 021 B1**
(45) Veröffentlichungstag und Bekanntmachung des Hinweises auf die Patenterteilung: **05.09.2018**
(21) Anmeldenummer: 12797808.8
(22) Anmeldetag: 21.11.2012
(51) Int. Cl.: A61Q 5/06, A61K 8/35, A61K 8/55

(54) **FÄRBEMITTEL MIT DIREKTZIEHENDEN FARBSTOFFEN UND PHOSPHAT-TENSIDEN**
COLORING AGENT COMPRISING DIRECT DYES AND PHOSPHATE SURFACTANTS
COMPOSITION DE COLORATION CONTENANT DES COLORANTS DIRECTS ET DES TENSIOACTIFS PHOSPHATES

(30) Priorität: 20.12.2011 DE 102011089221
(43) Veröffentlichungstag der Anmeldung: 29.10.2014
(73) Patentinhaber: Henkel AG & Co. KGaA, 40589 Düsseldorf (DE)
(72) Erfinder: WESER, Gabriele, 41472 Neuss (DE); KOLONKO, Claudia, 42857 Remscheid (DE)
(86) Internationale Anmeldenummer: PCT/EP2012/073247
(87) Internationale Veröffentlichungsnummer: WO 2013/092087

(56) Entgegenhaltungen:
- EP-A1- 1 820 826
- EP-A1- 2 329 809
- CA-A1- 2 613 049
- CA-A1- 2 646 866

## Beschreibung

Die vorliegende Erfindung betrifft Mittel zum Färben und gegebenenfalls Aufhellen von keratinischen Fasern, insbesondere menschlichen Haaren, welche bestimmte kationische Anthrachinonfarbstoffe und Phosphat-Tenside enthalten. Weiterhin betrifft die Erfindung die Verwendung dieser Mittel zur Erzeugung von Haarfärbungen mit erhöhtem Glanz, intensivem Farbergebnis, verbesserten Echtheitseigenschaften sowie verringerter Selektivität.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser. Solche Färbungen sind auch gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine sehr unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäsche können die Farbstoffe in den unterschiedlichen Haarbereichen verschieden stark auswaschen werden, wodurch ein uneinheitliches und damit unerwünschtes Farbergebnis erzielt wird.

Die Herstellung von Färbeformulierungen mit verringerter Selektivität, mittels welcher auf den unterschiedlich stark geschädigten Partien der Haare ein einheitliches Farbergebnis erzielt werden kann, steht bei der Entwicklung Färbeprodukten auf Basis von Direktziehern nach wie vor besonders im Fokus. Insbesondere soll diese verringerte Selektivität nicht nur direkt nach dem Färbeprozess, sondern auch nach wiederholten Haarwäschen noch vorhanden sein.
Die Aufgabe der vorliegenden Erfindung ist daher die Bereitstellung eines Färbemittels für keratinische Fasern, insbesondere menschliche Haare, welches neben anderen positiven Echtheitseigenschaften insbesondere eine geringe Selektivität (bzw. ein gutes Egalisiervermögen) und eine gute Waschechtheit besitzt.
Die mit den erfindungsgemäßen Mitteln erzeugten Färbungen sollen sowohl direkt nach dem Färbevorgang als auch nach wiederholten Haarwäschen ein brillantes und intensives Farbergebnis liefern. Hierbei sollen die Haare nach Anwendung des Färbemittels auch bei Vorhandensein von Haaren unterschiedlichen Schädigungsgrades gleichmäßig gefärbt sein, wobei diese Gleichmäßigkeit im Farbergebnis auch nach wiederholten Haarwäschen noch vorhanden sein soll. Insbesondere sollen brillante und neutrale Blaunuancen bzw. Nuancen im Blaubereich mit den oben beschriebenen vorteilhaften Echtheitseigenschaften erzielt werden, welche sich hervorragend auch für die Mattierung eigenen. Zusätzlich sollen die Mittel eine sowohl im Hinblick auf den Anwendungsprozess als auch auf die Färbeleistung optimierte Viskosität besitzen.
Der Einsatz von kationischen Anthrachinonfarbstoffen in Produkten zum Färben von keratinischen Fasern ist prinzipiell bereits aus dem Stand der Technik, beispielsweise aus EP 1 006 154 B1, CA 2 613 049 A1 oder aus EP 1 820 826 A1, bekannt. Ferner werden in EP 2 329 809 Kombinationen von kationischen Anthrachinonfarbstoffen mit Oxidationsfarbstoffvorprodukten vom Entwicklertyp für die oxidative Färbung von Haaren beansprucht.
Überraschend hat sich gezeigt, dass Kombinationen aus bestimmten kationischen Anthrachinonen als direktziehenden Farbstoffen mit Phosphat-Tensiden zu Färbungen führen, welche die Aufgabenstellung in herausragendem Maß lösen.
Die Kombinationen aus den bestimmten kationischen Anthrachinone gemäß erstem Erfindungsgegenstand mit Phosphat-Tensiden sind im Stand der Technik bislang noch nicht beschrieben.
Ein erster Gegenstand der vorliegenden Erfindung ist ein Mittel zum Färben und gegebenenfalls Aufhellen von keratinischen Fasern, insbesondere menschlichen Haaren, welches dadurch gekennzeichnet ist, dass es in einem kosmetischen Träger
(a) mindestens eine Verbindung der Formel (Ia) und
(b) mindestens ein Phosphat-Tensid enthält.
Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.
Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.
Die erfindungsgemäßen Mittel enthalten die Wirkstoffe in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tablettenförmige Formulierung zu integrieren.
Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. wässriger Träger enthält im Sinne der Erfindung mindestens 30 Gew.-%, insbesondere mindestens 50 Gew.-% Wasser, bezogen auf das Gesamtgewicht der Mittel. Erfindungsgemäß bevorzugt sind wässrige Träger, so dass das Mittel einen Anteil von mindestens 80 Gew.-%, bevorzugt mindestens 85 Gew.-%, bezogen auf das Gesamtgewicht des Mittels aufweist.
Als ersten wesentlichen Inhaltsstoff (a) enthalten die erfindungsgemäßen Mittel mindestens einen direktziehenden kationischen Anthrachinonfarbstoff der Formel (Ia). Optimal geeignet zur Lösung der erfindungsgemäßen Aufgabenstellung war die Verbindung der Formel (Ia), 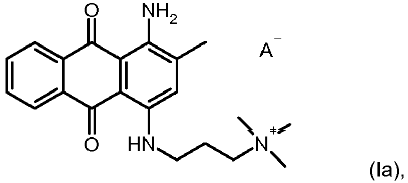 worin A⁻ für ein physiologisch verträgliches Anion, bevorzugt für Methylsulfat (H₃COSO₃⁻), steht. Die Verbindung (Ia), bei der A⁻ für Methylsulfat steht, ist auch unter der Bezeichnung Cationic Blue 347 bekannt.

Die erfindungsgemäßen Mittel zum Färben und gegebenenfalls gleichzeitigen Aufhellen von Keratinfasern enthalten die Verbindung(en) der Formel (Ia) vorzugsweise in Mengen oberhalb von 0,0001 Gew.-% und unterhalb von 5 Gew.-%, jeweils bezogen auf das gesamte Mittel.
Eine weitere bevorzugte Ausführungsform ist dabei ein Mittel, welches die Verbindung(en) der Formel (Ia) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.
Als zweiten wesentlichen Formulierungsbestandteil (b) enthalten die erfindungsgemäßen Mittel mindestens ein Phosphat-Tensid,
Als Phosphat-Tenside werden Verbindungen bezeichnet, die neben mindestens einem hydrophoben Molekülteil, zumeist langkettigen Alkylresten, einen hydrophilen Teil aufweisen, der eine Phosphat-Gruppe sowie gegebenenfalls Polyethylenoxid und/oder Polypropylenoxid-Einheiten aufweist.
Als hydrophober Teil kommen bevorzugt C₈-C₃₀-Alkylreste, besonders bevorzugt C₁₂-C₂₄-Alkylreste, zum Einsatz, welche gesättigt oder ungesättigt sowie gegebenenfalls verzweigt sein können. Je nach Herkunft und Herstellung ist es bevorzugt, als Phosphat-Tensid ein Gemisch aus Verbindungen mit unterschiedlicher Alkylkettenlänge einzusetzen.
Die Phosphat-Tenside können entweder in ihrer protonierten Form, also sauren Form, eingesetzt werden oder in Form von entsprechenden Phosphat-Salzen, wobei als geeignete Gegenionen Alkaliumetallionen, Ammoniumoinen, Mono-/Di- oder Tri-Alkylammoniumionen sowie Mono-/Di-oder Tri-(Hydroxyalkyl)ammoniumionen bevorzugt sind. Besonders bevorzugte Phosphat-Tenside werden als freie Säure oder als Natrium-Salz, Ammonium-Salz, Ethanolammonium-Salz (MEA), Diethanolammonium-Salz (DEA) oder Triethanolammonium-Salz (TEA) eingesetzt.

Der hydrophile Teil des Phosphat-Tensids kann neben der Phosphat-Gruppe gegebenenfalls Polyethylenoxid und/oder Polypropylenoxid-Einheiten umfassen. Dabei sind Phosphat-Tenside bevorzugt, die 1 bis 15, bevorzugt 3 bis 10 Ethylenoxid-Einheiten und/oder 1 bis 10, bevorzugt 3 bis 8 Propylenoxid-Einheiten aufweisen.

Eine weitere Ausführungsform des ersten Erfindungsgegenstands ist daher ein Mittel, welches dadurch gekennzeichnet ist, dass es als Phosphat-Tensid mindestens eine Verbindung, ausgewählt aus C₈-C₃₀-Alkyl-phosphat, C₈-C₃₀-Alkylether-phosphat, Di(C₈-C₃₀-Alkyl)-phosphat und/oder Gemischen daraus, bevorzugt ausgewählt aus C₁₂-C₂₄-Alkyl-phosphat, C₁₂-C₂₄-Alkylether-phosphat, Di(C₁₂-C₂₄-Alkyl)-phosphat und/oder Gemischen daraus, enthält.

Beispiele für geeignete C₈-C₃₀-Alkyl-phosphate sind Decyl Phosphate, Lauryl Phosphate, C₁₂-C₁₃-Alkyl Phosphatee, Myristyl Phosphate, Cetyl Phosphate, Stearyl Phosphate, Isostearyl Phosphate, Cetearyl Phosphate, Oleyl Phosphate und Behenyl Phosphate.

Beispiele für geeignete C₈-C₃₀-Alkylether-phosphate sind Deceth-3 Phosphate, Deceth-4 Phosphate, Deceth-5 Phosphate, Deceth-8 Phosphate, Deceth-10 Phosphate, Deceth-12 Phosphate, Deceth-15 Phosphate, Laureth-3 Phosphate, Laureth-4 Phosphate, Laureth-5 Phosphate, Laureth-8 Phosphate, Laureth-10 Phosphate, Laureth-12 Phosphate, Laureth-15 Phosphate, Ceteth-3 Phosphate, Ceteth-4 Phosphate, Ceteth-5 Phosphate, Ceteth-8 Phosphate, Ceteth-10 Phosphate, Ceteth-12 Phosphate, Ceteth-15 Phosphate, Steareth-3 Phosphate, Steareth-4 Phosphate, Steareth-5 Phosphate, Steareth-8 Phosphate, Steareth-10 Phosphate, Steareth-12 Phosphate, Steareth-15 Phosphate, Ceteareth-3 Phosphate, Ceteareth-4 Phosphate, Ceteareth-5 Phosphate, Ceteareth-8 Phosphate, Ceteareth-10 Phosphate, Ceteareth-12 Phosphate, Ceteareth-15 Phosphate, Oleth-3 Phosphate, Oleth-4 Phosphate, Oleth-5 Phosphate, Oleth-8 Phosphate, Oleth-10 Phosphate, Oleth-12 Phosphate, Oleth-15 Phosphate sowie PPG-3-Ceteth-10 Phosphate, PPG-3-Ceteth-15 Phosphate, PPG-5-Ceteth-10 Phosphate, PPG-5-Ceteth-15 Phosphate, PPG-3-Oleth-10 Phosphate, PPG-3-Oleth-15 Phosphate, PPG-5-Oleth-10 Phosphate und PPG-5-Oleth-15 Phosphate.

Beispiele für geeignete Di(C₈-C₃₀-alkyl)-phosphate sind Didecyl Phosphate, Dilauryl Phosphate, Di(C₁₂-C₁₃-Alkyl) Phosphatee, Dimyristyl Phosphate, Dicetyl Phosphate, Distearyl Phosphate, Diisostearyl Phosphate, Dicetearyl Phosphate, Dioleyl Phosphate und Dibehenyl Phosphate.

Ganz besonders vorteilhafte Ergebnisse im Hinblick auf Farbaufzug und Pflegewirkung an der Faser werden mit Gemischen aus C₈-C₃₀-Alkylether-phosphat und Di(C₈-C₃₀-Alkyl)-phosphat erzielt. Besonders vorteilhafte Phosphat-Tensid sind daher Gemische aus C₈-C₃₀-Alkylether-phosphat und Di(C₈-C₃₀-Alkyl)-phosphat.

Beispiele für besonders bevorzugte Phosphat-Tenside sind die Gemische aus Oleth-5 Phosphate und Dioleyl Phosphate sowie aus Dicetyl Phosphate und Ceteth-10 Phosphate.

Eine weitere Ausführungsform des ersten Erfindungsgegenstands ist daher ein Mittel, welches dadurch gekennzeichnet ist, dass es als Phosphat-Tensid ein Gemisch aus mindestens einem C₈-C₃₀-Alkylether-phosphat und mindestens einem Di(C₈-C₃₀-Alkyl)-phosphat, bevorzugt ein Gemisch aus mindestens einem C₁₂-C₂₄-Alkylether-phosphat und mindestens einem Di(C₁₂-C₂₄-Alkyl)-phosphat, enthält.

Die Phosphat-Tenside sind im Mittel bevorzugt in einem Anteil vorhanden, der eine optimale Pflegeleistung der Haare garantiert, die Löslichkeit oder die Emulgierbarkeit weiterer Inhaltsstoffe entsprechend unterstützt und die Viskosität der Mittel einstellt. Andererseits sollte der Gehalt an Phosphat-Tensiden möglichst gering gehalten sein, um Rohstoffe einzusparen und einem übermäßigen Entfetten der Haut vorzubeugen.

Eine weitere Ausführungsform des ersten Erfindungsgegenstands ist daher ein Mittel, welches dadurch gekennzeichnet ist, dass es das oder die Phosphat-Tensid(e) in einem Anteil von 0,01 bis 15 Gew.-%, bevorzugt 0,05 bis 12 Gew.-%, besonders bevorzugt 0,1 bis 10,0 Gew.-%, insbesondere 0,5 bis 8,0 Gew.-%, und insbesondere bevorzugt von 1,0 bis 7,5 Gew.-%, jeweils bezogen auf das Gesamtgewicht des Mittels, enthält.

In einer weiteren bevorzugten Ausführungsform enthalten die erfindungsgemäßen Mittel neben der Verbindung der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Färbeergebnisse mit herausragender Farbintensität, Brillanz und guter Waschechtheit werden insbesondere dann erhalten, wenn die erfindungsgemäßen Mittel als weiteren direktziehenden Farbstoff mindestens einen Farbstoff ausgewählt aus D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthalten.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxy-ethylamino)-1-methylbenzol, 2-({3-[(2-Hydroxyethyl)amino]-4-methoxy-5-methylphenyl}amino)-ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]-ethanol, 3-Amino-4-(2-methoxyethoxy)-5-methylphenylamin, 1-Amino-3-bis-(2-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die direktziehenden Farbstoffe, Entwicklerkomponenten und Kupplerkomponenten werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die Mittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen.

Eine weitere Ausführungsform des ersten Erfindungsgegenstands ist daher dadurch gekennzeichnet, dass das Mittel zusätzlich Wasserstoffperoxid und/oder eines seiner festen Anlagerungsprodukte an organische oder anorganische Verbindungen enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Eine weitere bevorzugte Ausführungsform ist ein Mittel zum Färben und gegebenenfalls Aufhellen keratinischer Fasern, welches zusätzlich Wasserstoffperoxid, eines seiner festen Anlagerungsprodukte an organische oder anorganische Verbindungen, Ammoniumperoxodisulfat, Kaliumperoxodisulfat und/oder Natriumperoxodisulfat jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, enthält.

Das Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus weiteren anionischen, kationischen, nichtionischen, amphoteren und zwitterionischen Tensiden und Emulgatoren ausgewählt.

Erfindungsgemäß bevorzugte Mittel sind dadurch gekennzeichnet, dass das Mittel neben den Phosphat-Tensiden zusätzlich mindestens ein weiteres anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die zusätzlichen anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Als nichtionische Tenside eignen sich insbesondere Anlagerungsprodukte von Ethylenoxid und/oder Propylenoxid an Fettalkohole, Fettsäuren, Fettsäureamide, Polyolester von Fettsäuren sowie und Polyolether von Fettalkoholen sowie Alkylpolyglucoside. Beispiele für geeignete nichtionische Tenside sind Laureth-2, Beheneth-10, Ceteareth-12, Trideceth-12, Oleth-16, Ceteareth-20, Ceteareth-30 und Ceteareth-50 sowie PPG-1 Trideceth-6, PEG-7 Oleate, PEG-90 Stearate, PEG-30 Cocoate, Polysorbate-20, Polysorbate-60, Polysorbate-65, Polysorbate-80, Polysorbate-85, Lauryl Glucoside, Decyl Glucoside und/oder Coco Glucoside.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Die nichtionischen, zwitterionischen oder amphoteren Tenside werden in Anteilen von 0,01 bis 45 Gew.-%, bevorzugt 0,1 bis 30 Gew.-% und ganz besonders bevorzugt von 1 bis 15 Gew.-%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Mittel können weitere Hilfs- und Zusatzstoffe enthalten. So hat es sich als vorteilhaft erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- kationische, synthetische Polymere;
- anionische, synthetische Polymere, wie Polyacrylate, Acrylates Copolymer, Copolymere von Acrylsäure und Methacrylsäure
- natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Xanthane, Alginate, Stärke-Fraktionen und Derivate wie Amylose, Amylopektin und Dextrine, sowie Cellulosederivate, wie beispielsweise Carboxymethylcellulose Methylcellulose und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im schwach sauren bis alkalischen Bereich, bevorzugt im schwach sauren bis schwach alkalischen Milieu ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 3 und 11, bevorzugt zwischen 5 und 8 liegen. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel werden bevorzugt aus Ammoniak, Alkanolaminen, basischen Aminosäuren sowie anorganischen Alkalisierungsmitteln ausgewählt. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.. Zur Einstellung des pH-Werts verwendbare Acificierungsmittel sind organische Säuren, wie Citronensäure, Essigsäure, Ascorbinsäure, Benzoesäure, Milchsäure, Äpfelsäure und Maleinsäure, sowie mineralische Säuren, wie Salzsäure, Schwefelsäure oder Phosphorsäure.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Poly-oxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol, insbesondere Polyquaternium-2, Polyquaternium-4, Polyquaternium-6, Polyquaternium-7, Polyquaternium-10, Polyquaternium-11, Polyquaternium-16, Polyquaternium-24, Polyquaternium-28, Polyquaternium-37, Polyquaternium-44, Polyquaternium-46, Polyquaternium-55, Polyquaternium-67, Polyquaternium-68, Polyquaternium-69 und Polyquaternium-87; zwitterionische und amphotere Polymere, wie insbesondere Polyquaternium-22 und Polyquaternium-39; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben und gegebenenfalls Aufhellen von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Färbemittel einen stark tensidhaltigen Träger besitzt.
Die erfindungsgemäßen Mittel können als Einkomponentenmittel (Färbe- und Aufhellmittel) oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.
Enthält das erfindungsgemäße Mittel sowohl direktziehende Farbstoffe - sowie gegebenenfalls zusätzlich Oxidationsfarbstoffvorprodukte - als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.
Ein Färbeverfahren, bei dem die Färbecreme und das Oxidationsmittel zunächst getrennt vorliegen, ist daher bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel enthaltend mindestens eine Verbindung der Formel (Ia) zu einer homogenen Zusammensetzung vermischt, und diese auf das Haar aufgebracht wird. Das Phosphat-Tensid (b) kann in diesem Fall sowohl zusammen mit der Wasserstoffperoxid-Lösung als auch mit der Verbindung der Formel (Ia) konfektioniert werden.
In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens einen kationischen Anthrachinonfarbstoff der Formel (Ia) und mindestens ein Phosphat-Tensid (b) - sowie gegebenenfalls zusätzlich Oxidationsfarbstoffvorprodukte enthält, und ein weiterer Container eine Oxidationsmittelzubereitung (B), enthaltend mindestens ein Oxidationsmittel, enthält.

Die Formulierung einer Kombination von (a) Verbindungen der Formel (Ia) mit (b) den Phosphat-Tensiden eignet sich hervorragend zur Erzeugung von intensiven Färbungen mit hoher Brillanz, hohem Glanz und einer niedrigen Selektivität in Verbindung mit einer hervorragenden Waschechtheit.

Bezüglich weiterer bevorzugter Ausführungsformen der erfindungsgemäßen Verfahren gilt mutatis mutandis das zu den erfindungsgemäßen Mitteln Gesagte.

### Beispiele

| **Formulierungsbeispiel 1** | **Gew.-%** |
|---|---|
| Propylene carbonate | 10,0 |
| Ethanol, 96 % | 43,0 |
| D&C Red No.33 | 0,05 |
| Acid Black No. 1 | 0,005 |
| D&C Orange No. 4 | 0,20 |
| Acid Violet 43 | 0,030 |
| Acid Red 18 | 0,20 |
| Crodafos HCE-LQ | 1,00 |
| SME 253 | 3,50 |
| Malic Acid | 0,40 |
| Cationic Blue 347 | 0,20 |
| Parfum | qs |
| Wasser | Ad 100 |

| | |
|---|---|
| Cationic Blue 347 | 3-[(4-Amino-9,10-dihydro-3-methyl-9,10-dioxo-1-anthracenyl)amino]-N,N,N-trimethyl-1-propanaminiummethylsulfat |
| Crodafos HCE-LQ | INCI-Bezeichnung: Oleth-5 Phosphate, Dioleyl Phosphate (Croda) |
| SME 253 | ca. 26-33% Aktivsubstanz; INCI-Bezeichnung: Amodimethicone, C11-15 Pareth-7, Laureth-9, Glycerin, Trideceth-12 (Bayer Silicones) |

## Patentansprüche

1. Mittel zum Färben und/oder Aufhellen von keratinischen Fasern, insbesondere menschlichen Haaren, **dadurch gekennzeichnet, dass** es in einem kosmetischen Träger
(a) mindestens eine Verbindung der Formel (Ia) 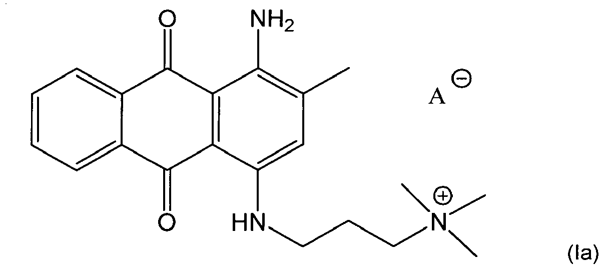 worin
A⁻ für ein physiologisch verträgliches Anion steht, und
(b) mindestens ein Phosphat-Tensid enthält.

2. Mittel nach Anspruch 1, **dadurch gekennzeichnet, dass** es die Verbindung(en) gemäß Formel (Ia) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

3. Mittel nach einem der Ansprüche 1 bis 2, **dadurch gekennzeichnet, dass** es als Phosphat-Tensid mindestens ein C₈-C₃₀-Alkyl-Phosphat, ein C₈-C₃₀-Alkylether-Phosphat, ein Di(C₈-C₃₀-Alkyl)-Phosphat und/oder Gemische daraus enthält.

4. Mittel nach einem der Ansprüche 1 bis 3, **dadurch gekennzeichnet, dass** es als Phosphat-Tensid ein Gemisch aus mindestens einem C₈-C₃₀-Alkylether-Phosphat und mindestens einem Di(C₈-C₃₀-Alkyl)-Phosphat enthält.

5. Mittel nach einem der Ansprüche 1 bis 4, **dadurch gekennzeichnet, dass** es das oder die Phosphat-Tensid(e) in einem Anteil von 0,01 bis 15 Gew.-%, bevorzugt 0,05 bis 10 Gew.-%, besonders bevorzugt 0,1 bis 5,0 Gew.-%, insbesondere 0,2 bis 3,5 Gew.-%, und insbesondere bevorzugt von 0,5 bis 2,5 Gew.-%, jeweils bezogen auf das Gesamtgewicht des Mittels, enthält.

6. Mittel nach einem der Ansprüche 1 bis 5, **dadurch gekennzeichnet, dass** es als Phosphat-Tensid(e) mindestens eine Verbindung aus der Gruppe Decyl Phosphate, Lauryl Phosphate, C₁₂-C₁₃-Alkyl Phosphate, Myristyl Phosphate, Cetyl Phosphate, Stearyl Phosphate, Isostearyl Phosphate, Cetearyl Phosphate, Oleyl Phosphate und Behenyl Phosphate enthält.

7. Mittel nach einem der Ansprüche 1 bis 6, **dadurch gekennzeichnet, dass** es als Phosphat-Tensid(e) mindestens eine Verbindung aus der Gruppe Deceth-3 Phosphate, Deceth-4 Phosphate, Deceth-5 Phosphate, Deceth-8 Phosphate, Deceth-10 Phosphate, Deceth-12 Phosphate, Deceth-15 Phosphate, Laureth-3 Phosphate, Laureth-4 Phosphate, Laureth-5 Phosphate, Laureth-8 Phosphate, Laureth-10 Phosphate, Laureth-12 Phosphate, Laureth-15 Phosphate, Ceteth-3 Phosphate, Ceteth-4 Phosphate, Ceteth-5 Phosphate, Ceteth-8 Phosphate, Ceteth-10 Phosphate, Ceteth-12 Phosphate, Ceteth-15 Phosphate, Steareth-3 Phosphate, Steareth-4 Phosphate, Steareth-5 Phosphate, Steareth-8 Phosphate, Steareth-10 Phosphate, Steareth-12 Phosphate, Steareth-15 Phosphate, Ceteareth-3 Phosphate, Ceteareth-4 Phosphate, Ceteareth-5 Phosphate, Ceteareth-8 Phosphate, Ceteareth-10 Phosphate, Ceteareth-12 Phosphate, Ceteareth-15 Phosphate, Oleth-3 Phosphate, Oleth-4 Phosphate, Oleth-5 Phosphate, Oleth-8 Phosphate, Oleth-10 Phosphate, Oleth-12 Phosphate, Oleth-15 Phosphate, PPG-3-Ceteth-10 Phosphate, PPG-3-Ceteth-15 Phosphate, PPG-5-Ceteth-10 Phosphate, PPG-5-Ceteth-15 Phosphate, PPG-3-Oleth-10 Phosphate, PPG-3-Oleth-15 Phosphate, PPG-5-Oleth-10 Phosphate und PPG-5-Oleth-15 Phosphate enthält.

8. Mittel nach einem der Ansprüche 1 bis 7, **dadurch gekennzeichnet, dass** es als Phosphat-Tensid(e) mindestens eine Verbindung aus der Gruppe Didecyl Phosphate, Dilauryl Phosphate, Di(C₁₂-C₁₃-Alkyl) Phosphate, Dimyristyl Phosphate, Dicetyl Phosphate, Distearyl Phosphate, Diisostearyl Phosphate, Dicetearyl Phosphate, Dioleyl Phosphate und Dibehenyl Phosphate enthält.

9. Mittel nach einem der Ansprüche 1 bis 8, **dadurch gekennzeichnet, dass** es als Phosphat-Tensid(e) ein Gemisch aus Oleth-5 Phosphate und Dioleyl Phosphate enthält.

10. Mittel nach einem der Ansprüche 1 bis 9, **dadurch gekennzeichnet, dass** es als Phosphat-Tensid(e) ein Gemisch aus Dicetyl Phosphate und Ceteth-10 Phosphate enthält.

11. Verfahren zum Färben menschlicher Haare, umfassend die Schritte:
A. Auftragen eines Mittel nach einem der Ansprüche 1 bis 5 auf das Haar;
B. Einwirken des Mittels für einen Zeitraum von 5 bis 45 Minuten, bevorzugt von 10 bis 35 Minuten, auf dem Haar;
C. Ausspülen des Haares.

## Claims

1. An agent for dyeing and/or lightening keratin fibers, in particular human hair, **characterized in that** it contains, in a cosmetic carrier,
(a) at least one compound of formula (Ia) 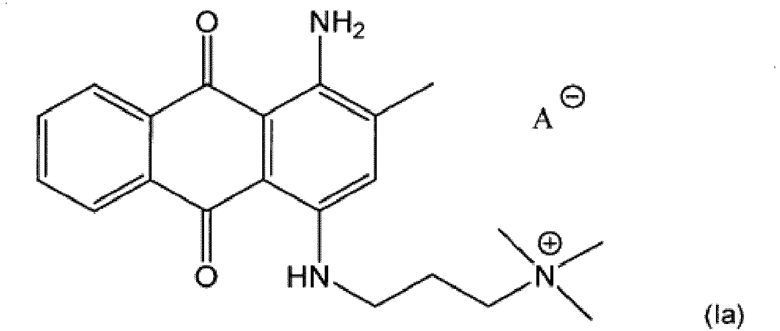 where
A⁻ represents a physiologically acceptable anion, and
(b) at least one phosphate surfactant.

2. The agent according to claim 1, **characterized in that** it contains the compound(s) according to formula (Ia) in a proportion of from 0.0001 to 5 wt.%, preferably from 0.005 to 3.5 wt.%, particularly preferably from 0.01 to 2.5 wt.%, in particular from 0.05 to 1.5 wt.%, and more particularly preferably from 0.01 to 1.0 wt.%, in each case based on the total weight of the agent.

3. The agent according to one of claims 1 to 2, **characterized in that** it contains, as the phosphate surfactant, at least one C₈-C₃₀ alkyl phosphate, C₈-C₃₀ alkyl ether phosphate, Di(C₈-C₃₀ alkyl) phosphate and/or mixtures thereof.

4. The agent according to one of claims 1 to 3, **characterized in that** it contains, as the phosphate surfactant, a mixture of at least one C₈-C₃₀ alkyl ether phosphate and at least one Di(C₈-C₃₀ alkyl) phosphate.

5. The agent according to one of claims 1 to 4, **characterized in that** it contains the phosphate surfactant(s) in a proportion of from 0.01 to 15 wt.%, preferably from 0.05 to 10 wt.%, particularly preferably from 0.1 to 5.0 wt.%, in particular from 0.2 to 3.5 wt.%, and more particularly preferably from 0.5 to 2.5 wt.%, in each case based on the total weight of the agent.

6. The agent according to one of claims 1 to 5, **characterized in that** it contains, as the phosphate surfactant(s), at least one compound from the group of decyl phosphate, lauryl phosphate, C₁₂-C₁₃ alkyl phosphate, myristyl phosphate, cetyl phosphate, stearyl phosphate, isostearyl phosphate, cetearyl phosphate, oleyl phosphate and behenyl phosphate.

7. The agent according to one of claims 1 to 6, **characterized in that** it contains, as the phosphate surfactant(s),
at least one compound from the group of Deceth-3 phosphate, Deceth-4 phosphate, Deceth-5 phosphate, Deceth-8 phosphate, Deceth-10 phosphate, Deceth-12 phosphate, Deceth-15 phosphate, Laureth-3 phosphate, Laureth-4 phosphate, Laureth-5 phosphate, Laureth-8 phosphate, Laureth-10 phosphate, Laureth-12 phosphate, Laureth-15 phosphate, Ceteth-3 phosphate, Ceteth-4 phosphate, Ceteth-5 phosphate, Ceteth-8 phosphate, Ceteth-10 phosphate, Ceteth-12 phosphate, Ceteth-15 phosphate, Steareth-3 phosphate, Steareth-4 phosphate, Steareth-5 phosphate, Steareth-8 phosphate, Steareth-10 phosphate, Steareth-12 phosphate, Steareth-15 phosphate, Ceteareth-3 phosphate, Ceteareth-4 phosphate, Ceteareth-5 phosphate, Ceteareth-8 phosphate, Ceteareth-10 phosphate, Ceteareth-12 phosphate, Ceteareth-15 phosphate, Oleth-3 phosphate, Oleth-4 phosphate, Oleth-5 phosphate, Oleth-8 phosphate, Oleth-10 phosphate, Oleth-12 phosphate, Oleth-15 phosphate, PPG-3-Ceteth-10 phosphate, PPG-3-Ceteth-15 phosphate, PPG-5-Ceteth-10 phosphate, PPG-5-Ceteth-15 phosphate, PPG-3-Oleth-10 phosphate, PPG-3-Oleth-15 phosphate, PPG-5-Oleth-10 phosphate and PPG-5-Oleth-15 phosphate.

8. The agent according to one of claims 1 to 7, **characterized in that** it contains, as the phosphate surfactant(s), at least one compound from the group of didecyl phosphate, dilauryl phosphate, Di(C₁₂-C₁₃ alkyl) phosphate, dimyristyl phosphate, dicetyl phosphate, distearyl phosphate, diisostearyl phosphate, dicetearyl phosphate, dioleyl phosphate and dibehenyl phosphate.

9. The agent according to one of claims 1 to 8, **characterized in that** it contains, as the phosphate surfactant(s), a mixture of Oleth-5 phosphate and dioleyl phosphate.

10. The agent according to one of claims 1 to 9, **characterized in that** it contains, as the phosphate surfactant(s), a mixture of dicetyl phosphate and Ceteth-10 phosphate.

11. A method for dyeing human hair, comprising the steps of:
A. applying an agent according to one of claims 1 to 5 to the hair;
B. allowing the agent to act on the hair for a period of from 5 to 45 minutes, preferably from 10 to 35 minutes;
C. rinsing the hair.

## Revendications

1. Agent de coloration et/ou d'éclaircissement de fibres kératiniques, en particulier de cheveux humains, **caractérisé en ce qu'**il contient, dans un agent cosmétique,
(a) au moins un composé de formule (Ia) 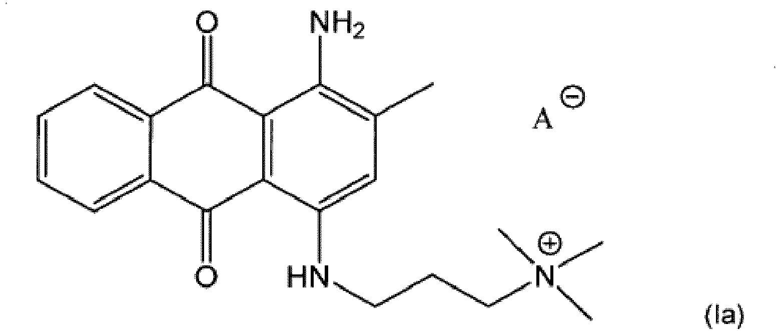 dans laquelle
A⁻ représente un anion physiologiquement compatible, et
(b) au moins un agent tensioactif phosphate.

2. Agent selon la revendication 1, **caractérisé en ce qu'**il contient le ou les composé(s) selon la formule (Ia) dans une proportion de 0,0001 à 5 % en poids, de préférence de 0,005 à 3,5 % en poids, de manière particulièrement préférée de 0,01 à 2,5 % en poids, en particulier de 0,05 à 1,5 % en poids et de manière particulièrement préférée de 0,01 à 1,0 % en poids, par rapport au poids total de l'agent.

3. Agent selon l'une des revendications 1 et 2, **caractérisé en ce qu'**il contient, comme agent tensioactif phosphate, au moins un phosphate alkyle en C₈-C₃₀, un alkyl éther phosphate en C₈-C₃₀, un diphosphate (alkyle en C₈-C₃₀) et/ou des mélanges de ceux-ci.

4. Agent selon l'une des revendications 1 à 3, **caractérisé en ce qu'**il contient, comme agent tensioactif phosphate, un mélange d'au moins un alkyl éther phosphate C₈-C₃₀ et au moins un diphosphate (alkyle en C₈-C₃₀).

5. Agent selon l'une des revendications 1 à 4, **caractérisé en ce qu'**il contient le ou les agent(s) tensioactif(s) phosphate dans une proportion de 0,01 à 15 % en poids, de préférence de 0,05 à 10 % en poids, de manière particulièrement préférée de 0,1 à 5,0 % en poids, en particulier de 0,2 à 3,5 % en poids et de manière tout particulièrement préférée de 0,5 % à 2,5 % en poids, par rapport au poids total de l'agent respectivement.

6. Agent selon l'une des revendications 1 à 5, **caractérisé en ce qu'**il contient, comme agent(s) tensioactif(s) phosphate, au moins un composé du groupe des décylphosphates, des laurylphosphates, des alkylphosphates en C₁₂-C₁₃, des myristylphosphates, des cétylphosphates, des stéarylphosphates, des isostéarylphosphates, des cétéarylphosphates, des oléylphosphates et des béhénylphosphates.

7. Agent selon l'une des revendications 1 à 6, **caractérisé en ce qu'**il contient, comme agent(s) tensioactif(s) phosphate, au moins un composé du groupe des Deceth-3 phosphates, Deceth-4 phosphates, Deceth-5 phosphates, Deceth-8 phosphates, Deceth-10 phosphates, Deceth-12 phosphates, Deceth-15 phosphates, Laureth-3 phosphates, Laureth-4 phosphates, Laureth-5 phosphates, Laureth-8 phosphates, Laureth-10 phosphates, Laureth-12 phosphates, Laureth-15 phosphates, Ceteth-3 phosphates, Ceteth-4 phosphates, Ceteth-5 phosphates, Ceteth-8 phosphates, Ceteth-10 phosphates, Ceteth-12 phosphates, Ceteth-15 phosphates, Steareth-3 phosphates, Steareth-4 phosphates, Steareth-5 phosphates, Steareth-8 phosphates, Steareth-10 phosphates, Steareth-12 phosphates, Steareth-15 phosphates, Ceteareth-3 phosphates, Ceteareth-4 phosphates, Ceteareth-5 phosphates, Ceteareth-8 phosphates, Ceteareth-10 phosphates, Ceteareth-12 phosphates, Ceteareth-15 phosphates, Oleth-3 phosphates, Oleth-4 phosphates, Oleth-5 phosphates, Oleth-8 phosphates, Oleth-10 phosphates, Oleth-12 phosphates, Oleth-15 phosphates, PPG-3-Ceteth-10 Phosphates, PPG-3-Ceteth-15 phosphates, PPG-5-Ceteth-10 phosphates, PPG-5-Ceteth-15 phosphates, PPG-3-Oleth-10 Phosphates, PPG-3-Oleth-15 phosphates, PPG-5-Oleth-10 phosphates et PPG-5-Oleth-15 phosphates.

8. Agent selon l'une des revendications 1 à 7, **caractérisé en ce qu'**il contient, comme agent(s) tensioactif(s) phosphate, au moins un composé du groupe des didécylphosphates, des dilaurylphosphates, des diphosphates (alkyle en C₁₂-C₁₃), des dimyristylphosphates, des dicétylphosphates, des distéarylphosphates, des diisostéarylphosphates, des dicétéarylphosphates, des dioléylphosphates et des dibéhénylphosphates.

9. Agent selon l'une des revendications 1 à 8, **caractérisé en ce qu'**il contient, comme agent(s) tensioactif(s) phosphate, un mélange d'Oleth-5 phosphates et de dioléylphosphates.

10. Agent selon l'une des revendications 1 à 9, **caractérisé en ce qu'**il contient, comme agent(s) tensioactif(s) phosphate, un mélange de dicétylphosphates et de Ceteth-10 phosphates.

11. Procédé de coloration des cheveux humains, comprenant les étapes suivantes :
A. appliquer un agent selon l'une des revendications 1 à 5 sur les cheveux ;
B. laisser agir l'agent pendant une durée de 5 à 45 minutes, de préférence de 10 à 35 minutes, sur les cheveux ;
C. rincer les cheveux.
